# EUROPÄISCHE PATENTANMELDUNG

(11) **EP 1 046 379 A1**
(43) Veröffentlichungstag der Anmeldung: **25.10.2000**
(21) Anmeldenummer: 99108028.4
(22) Anmeldetag: 23.04.1999
(51) Int. Cl.: A61C 13/20, A61C 13/00, A61K 6/06

(54) **Verfahren zur Herstellung von Zahnersatzstücken aus Glas**

(71) Anmelder: Letzner, Jürgen, 51399 Burscheid (DE)
(72) Erfinder: Letzner, Jürgen, 51399 Burscheid (DE)
(74) Vertreter: Godemeyer, Thomas, Dr.

(57) **Zusammenfassung**

Die Erfindung betrifft ein Verfahren zur Herstellung von Zahnersatzstücken aus Glas gekennzeichnet durch die folgenden Verfahrensschritte:
a)Anfertigen eines Modells des kranken Zahnes, der mit dem Zahnersatzstück ausgestattet werden soll,
b)Fixieren des Modells in einer Aufnahmevorrichtung,
c)Herstellung einer Glasschmelze und Einpressen der Glasschmelze in die Hohlräume des Modells,
d)Abkühlen des mit der Glasschmelze gefüllten Modells.

Ein weiterer Gegenstand der Erfindung sind Zahnersatzstücke aus Glas hergestellt nach dem erfindungsgemäßen Verfahren.

## Beschreibung

Gegenstand der Erfindung ist ein Verfahren zur Herstellung von Zahnersatzstücken aus Glas, insbesondere zur Verwendung als Füllungen, Inlays, Onlays, Verblendschalen oder Kronen. Ein weiterer Gegenstand der Erfindung sind Zahnersatzstücke, hergestellt nach diesem Verfahren.

Für Zahnersatzstücke werden üblicherweise im Stand der Technik Materialien wie Keramik, Amalgam, Gold und auch Kunststoff verwendet.

Insbesondere Amalgam ist preisgünstig und leicht verarbeitbar und wird daher in großem Umfange seit vielen Jahren eingesetzt. In medizinischen Publikationen ist in den letzten Jahren aber wiederholt darauf hingewiesen worden, daß Amalgam unter Umständen auch Gesundheitsschäden hervorrufen kann, insbesondere durch Freisetzung des in der Legierung vorhandenen Quecksilbers. Soweit möglich wird daher mittlerweile in der Zahnmedizin auf Amalgamfüllungen zunehmend verzichtet und viele Patienten lassen sich vorhandene Amalgamfüllungen durch andere Füllungen ersetzen.

Die als Ersatzstoffe verwendeten Materialien Gold und Keramik sind teuer und auch nur aufwendiger verarbeitbar als Amalgam. Im Vergleich zu Amalgam bietet insbesondere Keramik den Vorteil, daß es farblich den gesunden Zähnen angepaßt werden kann. Hierzu ist jedoch bei der Anfertigung von Zahnersatzstücken ein erheblicher Arbeitsaufwand nötig, so daß der Preis derartiger Zahnersatzstücke den von Zahnersatzstücken aus Gold noch übersteigt.

Insbesondere in vorderen Zahnbereichen aber auch im Bereich der hinteren Zähne wird Kunststoff eingesetzt. Ein Material, das ebenfalls gut verarbeitbar und preisgünstig ist. Die Haltbarkeit dieser Zahnersatzstücke aus Kunststoff ist jedoch zeitlich begrenzt. Zum einen weil die Kunststoffmaterialien eine geringere Härte aufweisen als beispielsweise Keramikmaterialien und zum anderen weil diese Kunststoffmaterialien Schrumpfungsprozessen unterliegen und damit bei den behandelten Zähnen Gefahr von Sekundärkaries auftreten kann.

Insbesondere die Anfertigung von Zahnersatzstücken aus keramischen Materialien wurde in jüngster Zeit weiter entwickelt, um die aufwendige Herstellungsprozedur zu vereinfachen und die Gefahr von Sekundärkaries durch Paßungenauigkeiten der Zahnersatzstücke zu vermeiden.

Bekannte Zahnersatzstücke aus keramischen Materialien weisen häufig einen mehrschichtigen Aufbau auf. Dieser entsteht durch Auftragen und Brennen einzelner Schichten in einem mehrstufigen Herstellungsprozeß.

So beschreibt beispielsweise die DE 44 19 015 A1 die Herstellung eines Zahnersatzkörpers aus glaskeramischem Material. Dieser Zahnersatzkörper besteht aus mehreren Schichten, nämlich einem Kern, auf dem eine Dentinfarbe und gegebenenfalls Korrekturmassen und Malfarben aufgetragen sind und einer diesen abdeckenden Blendschicht aus keramischem Schmelz- und Transparentmaterial.

Dabei wird der Dentinkörper des nachzubildenden Zahnes zunächst aus einer keramischen Formmasse hergestellt und gebrannt. Danach wird diese gebrannte Keramik auf dem den Dentinkörper nachbildenden Kern beschliffen. Auf diesen Kern wird die Dentinfarbe aufgetragen und eingebrannt. Nach Auftragung der Transparenz- und Schmelzmassen erfolgt ein weiterer Brand. Im Anschluß daran erfolgt die Formgebung mit den üblichen Diamantschleifern und Keramikschleifsteinen, wobei auch die Anpassung an den Grad der Abnutzung der natürlichen Nachbarzähne erfolgt.

Die EP 0 630 639 A1 beschreibt eine weitere dentalkeramische Masse mit einem mehrschichtigen Aufbau. Dabei wird auf einen Kern eine Opaker-Schicht und eine Dentin-/Schmelzschicht aufgebracht, wonach ein Brennen dieser Schichten erfolgt. Auch hier werden zur Herstellung der keramischen Zahnersatzstücke zunächst ein Kern hergestellt und gebrannt, dann mehrere Schichten aufgebracht, die ebenfalls gebrannt werden müssen.

Eine Verbesserung der Herstellung von glaskeramischen Zahnersatzstücken im Sinterverfahren wird in der DE 37 39 096 beschrieben. Hier wird ein besonders geeignetes feuerfestes Zahnstumpfmodellmaterial eingesetzt, bei dem keine Paßungenauigkeiten mehr auftreten und das daher zu einer exakteren Herstellung der Zahnersatzstücke führt.

Glas als Material für Zahnersatzstücke wird bisher nicht eingesetzt, da es bei der Verarbeitung von Glas in den üblichen Gießverfahren zu verstärkten Schrumpfungsprozessen kommt, die eine exakte Herstellung eines Zahnersatzstückes quasi nicht möglich machen. Es sind daher andere Prozesse notwendig, um Glas im Dentalbereich zu verarbeiten. Ein derartiger Stand der Technik wird beispielsweise beschrieben in der DE 39 39 831 A1. Diese Druckschrift bezieht sich auf eine biokompatible Glaskeramik, die ein mechanisch hochfestes biokompatibles kristallisiertes Glas enthält. Dieses Glas enthält im wesentlichen sowohl Kristalle aus Tektrakieselsäure-FluorGlimmergruppen als auch aus Calciumphosphatgruppen. Das kristallisierte Glas besitzt im wesentlichen natriumfreies Oxid und es kann bei einer zahnmedizinischen Behandlung als Zahnersatzmaterial verwendet werden. Gemäß den Beispielen dieser Druckschrift wird das entsprechende Zahnersatzmaterial in eine Gußform eingeblasen und der Glaskörper mittels einer Schleudergußmaschine geformt.

Die technische Aufgabe der Erfindung ist es, ein Verfahren zur Herstellung eines Zahnersatzstückes aus Glas zur Verfügung zu stellen, wobei der Herstellungsprozeß einfacher und damit preiswerter ist, ein haltbares Zahnersatzstück angefertigt wird, keine Paßungenauigkeiten auftreten und bezüglich des Werkstoffes keine gesundheitlichen Bedenken vorliegen.

Diese technische Aufgabe wird gelöst durch ein Verfahren zur Herstellung von Zahnersatzstücken aus Glas, gekennzeichnet durch die folgenden Verfahrensschritte:
a)Anfertigen eines Modells 1 des kranken Zahnes, der mit dem Zahnersatzstück ausgestattet werden soll,
b)Fixieren des Modells in einer Aufnahmevorrichtung 2,
c)Herstellung einer Glasschmelze und Einpressen der Glasschmelze in die Hohlräume des Modells 1,
d)Abkühlen des mit der Glasschmelze gefüllten Modells 1.

Es wurde gefunden, daß mit diesen Verfahrensschritten, insbesondere des Schrittes des Schmelzens und Einpressens der Glasschmelze in die Hohlräume des Modells Zahnersatzstücke hergestellt werden können, die paßgenau sind und ohne aufwendige Nachbearbeitung den Patienten eingesetzt werden können.

Weiterhin ist der Werkstoff Glas gesundheitlich völlig unbedenklich und wird bereits seit vielen Jahren in medizinischen Bereichen, beispielsweise für Glasaugen, eingesetzt. In einer bevorzugten Ausführungsform werden als Zahnersatzstücke Inlays, Onlays, Verblendschalen oder Kronen hergestellt. Es ist weiterhin bevorzugt, daß das Modell vor dem Einpressen der Glasschmelze auf Temperaturen von 200 bis 500 °C vorgeheizt wird. Die Preßstempeltemperatur beträgt 200 bis 500 °C, bevorzugt 300° C. Die Temperatur der Glasschmelze beträgt 800 bis 1100° C, die Temperatur der Aufnahmevorrichtung 2 200 bis 500°C.

Zur Herstellung der Glasschmelze wird bevorzugt ein Glasstab, ein Glasstab mit Verdickung am unteren Ende oder Haltemittel mit Glas am unteren Ende eingesetzt und das am unteren Ende befindliche Glas mit einem Brenner 7 oder einer anderen geeigneten Wärmequelle geschmolzen.

Das Erreichen des Schmelzpunktes kann prinzipiell subjektiv ermittelt werden. Es besteht aber auch die Möglichkeit, das Erreichen des Schmelzpunktes mit einem Infrarotmeßgerät 8 oder anderen Meßgeräten zu ermitteln. Die Temperatur der Glasschmelze sollte bevorzugt bei 800 bis 1100° C liegen. Um ein gleichmäßiges Schmelzen des Glases zu erzielen, ist es weiterhin bevorzugt, daß während des Erhitzens des Glases der Glasstab, der Glasstab mit Verdickung am unteren Ende oder das Haltemittel mit Glas am unteren Ende um die eigene Achse rotiert. Nachdem das Glas geschmolzen ist, wird das Verfahren weitergeführt dadurch, daß das Einpressen der Glasschmelze in das Modell erfolgt. Dabei wird bevorzugt ein Preßkopf 6 verwendet, der mit einem Preßstempel 4 versehen ist und der die Glasschmelze in die Hohlräume des Modells hineindrückt.

Der Preßstempel wird bevorzugt auf Temperaturen von 200 bis 500° C, vorzugsweise 300° C vorgeheizt. Die überschüssige Glasschmelze kann bevorzugt seitlich abgeführt werden oder wird in einem über dem Modell angeordneten Hohlraum gesammelt. Es ist weiterhin bevorzugt, daß die eingesetzten Modelle in ihren Hohlräumen einen Luftkanal aufweisen, so daß beim Einpressen der Glasschmelze die in den Hohlräumen vorhandene Luft entweichen kann. Hierdurch kommt es zu einer besseren Füllung der Hohlräume des Modells.

Es ist weiterhin bevorzugt, daß die Modelle langsam abgekühlt werden, um ein gleichmäßigeres Entspannen der Glasschmelze zu ermöglichen. Der Transformationspunkt (Tg) liegt für die verwendeten Glassorten bei 400 bis 600° C. Er sollte beim Abkühlen 30 bis 90 min, vorzugsweise 60 min eingehalten werden. Danach erfolgt eine Abkühlung von etwa 2 bis 4° C, vorzugsweise 3° C pro Minute.

Nachfolgend soll das Herstellungsverfahren zur Herstellung von Zahnersatzstücken aus Glas ausführlich beschrieben werden.

Das Verfahren wird hier beispielhaft für die Herstellung eines Inlays beschrieben. Es können jedoch auch Zahnersatzstükke für andere Anwendungen wie Füllungen, Onlays, Verblendschalen oder Kronen in derselben Weise hergestellt werden.

Zunächst wird nach Ausbohren des von Karies befallenen Zahns ein Gebißabdruck des Ober- und Unterkiefers als Silikonabdruck gefertigt. Zur Modellpräparation wird von diesem Silikonabdruck ein Gipsabdruck hergestellt. Der Abdruck des vom Zahnarzt vorbehandelten Zahns, der mit dem Inlay gefüllt werden soll, wird aus dem Gipsmodell herausgetrennt und die Hohlräume mit Luftkanälen versehen, so daß die beim Einpressen des Glases entweichende Luft nach unten aus dem Modell ausdringen kann.

Das Modell 1 wird dann in einer Aufnahmevorrichtung 2 auf Temperaturen von 200 bis 500 °C vorgeheizt. Als Glasmaterial dient ein in einer Verdickung auslaufender Glasstab 3, der in der Aufnahmevorrichtung 6 verschiebbar befestigt wird. Die Aufnahmevorrichtung 6 ist in der Lage, eine Preßwirkung auszuüben und den Glasstab in Rotation zu versetzen. Diese Aufnahmevorrichtung, hier als Preßkopf 6 bezeichnet, kann beispielsweise aus einem Tischbohrgerät bestehen. Beim Einsetzen durchläuft der Glasstab einen mit einer Mittelbohrung versehenen aufheizbar konzipierten Preßstempel 4 und eine in dem Preßkopf 6 eingesetzte Schlupfbuchse 5. Der Glasstab mit der Verdickung wird sodann in Drehbewegung um seine eigene Achse versetzt. Dabei wird das Glas am unteren Ende mit einem geeigneten Heizgerät, beispielsweise mit einem Brenner 7 bis zum Erreichen des Schmelzpunktes von 800 bis 1100° C aufgeheizt. Das Erreichen des Schmelzpunktes kann subjektiv ermittelt werden oder aber durch Einsatz eines Infrarotmeßgerätes. Wenn der Schmelzpunkt erreicht wird, wird der Preßvorgang durchgeführt. Die Drehung des Glasstabes wird gestoppt und die Glasschmelze wird durch den Preßkopf mittels des vorgeheizten Preßstempels mit einer Kraft von 10 bis 20 N auf das Modell gedrückt, so daß die Glasschmelze in die Hohlräume des Modells eindringen kann. Nach Abschluß des Preßvorgangs wird das ausgeformte Inlay bevorzugt in einem Muffelofen langsam abgekühlt, um eine gleichmäßige Entspannung der Glassubstanz zu erreichen.

Nach dem Abkühlen wird das Glasinlay aus dem Modell entnommen, gereinigt und zur weiteren manuellen Anpassung der Kauflächenfissur in üblicher Weise nachbearbeitet. Danach kann das Glasinlay dem Patienten eingesetzt werden.

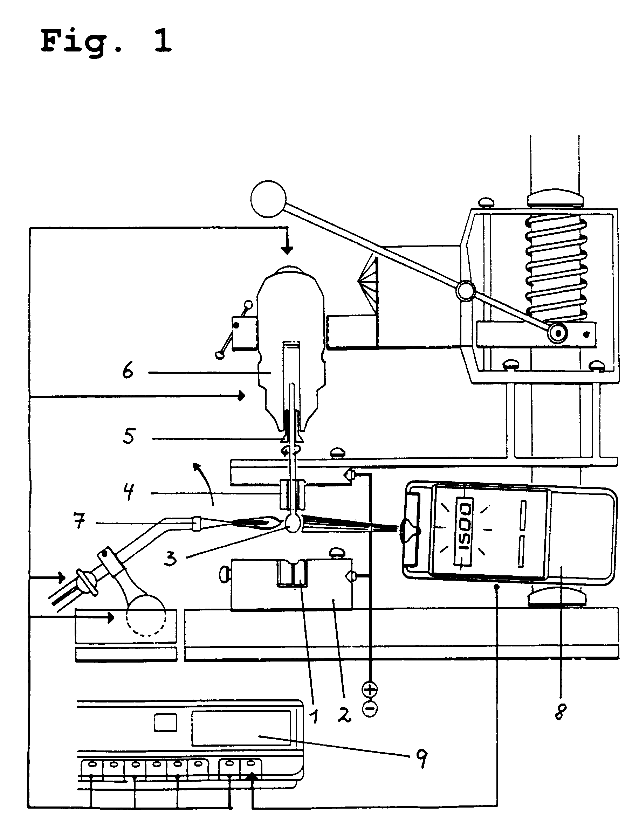

Die beigefügte Figur 1 zeigt ein Verfahrensablaufschema des erfindungsgemäßen Verfahrens. Die Ziffer 1 bezeichnet das Modell mit dem Luftkanal in der Aufnahmevorrichtung 2, die bevorzugt heizbar ist. Mit der Ziffer 3 ist der Glasstab mit der am unteren Ende liegenden Verdickung bezeichnet. Die Ziffer 4 beschreibt den ebenfalls aufheizbaren Preßstempel und die Ziffer 5 die Schlupfbuchse. Mit der Ziffer 6 ist ein Tischbohrgerät bezeichnet, das den Glasstab in Rotation versetzen kann und gleichzeitig die Funktion des Preßkopfes übernimmt. Ziffer 7 bezeichnet den Brenner, mit dem die Glasmasse erhitzt wird. Es kann sich hierbei um einen Gasbrenner aber auch um andere geeignete Heizgeräte handeln. Mit der Ziffer 8 wird ein IR-Meßgerät bezeichnet, um den Schmelzpunkt des Glases, der bei etwa 1100 °C liegt, zu ermitteln. Die Ziffer 9 bezeichnet die gesamte Steuerung der Anlage.

Mit dem erfindungsgemäßen Verfahren werden gegenüber herkömmlichen Zahnersatzstücken aus anderen Materialien zahlreiche Vorteile erreicht. Bei Glas handelt es sich um einen in der Medizin erprobten Werkstoff. So werden beispielsweise auch Glasaugen aus diesem Material hergestellt. Glas verhält sich gesundheitsneutral und weist keinerlei Risiken auf, wie beispielsweise Amalgam als Zahnersatzmaterial.

Das erfindungsgemäßen Schmelzpreßverfahren führt zu einer hohen Paßgenauigkeit des Zahninlays, so daß auftretender Sekundärkaries vermieden werden kann.

Das Herstellungsverfahren kann weitgehend automatisiert werden, wodurch sich die hohen Personalkosten, die bei anderen Verfahren, bei denen feste Materialien eingesetzt werden, erheblich reduzieren lassen. Auch die Materialkosten sind bei dem Einsatz von Glas äußerst gering. Die Anwendung kann daher auf dem Gebiet der Zahnersatztechnik sinnvoll verwendet werden und führt zu einer wirtschaftlichen Nutzung, die erheblich preiswerter und einfacher ist als bei den bisher verwendeten Zahnersatzmaterialien.

Ein weiterer Gegenstand der Erfindung ist ein Zahnersatzkörper aus Glas, hergestellt nach dem erfindungsgemäßen Verfahren. Dieser Zahnersatzkörper ist bevorzugt eine Füllung, Inlay, Onlay, eine Verblendung oder eine Krone und besteht aus Glas, vorzugsweise Silikatglas.

Glas besitzt den Vorteil, daß es Eigenschaften aufweist, die für die Verwendung als Zahninlay nahezu ideal sind. Es ist bißfest, besonders haltbar ohne zeitlich bedingte Schrumpfungsprozesse und weist nur einen sehr geringen Abrieb auf. Wie Keramik kann es farblich den gesunden Zähnen angepaßt werden durch Beimischung entsprechender Pigmente. Damit ist Glas mit dem natürlichen Zahnschmelz vergleichbar. Es besitzt beispielsweise auch gegenüber Keramik nur eine äußerst geringe Kontraktion, so daß eine präzise Paßgenauigkeit beim Füllen der Hohlräume des Modells erreicht werden kann. Dadurch wird der Verarbeitungsvorgang erheblich verkürzt, da eine Nachbearbeitung zum Ausgleich der Kontraktion wie bei üblichen keramischen Materialien nicht mehr notwendig ist. Dies führt zu einer beträchtlichen Kostenreduktion.

In einer bevorzugten Ausführungsform besitzt das Glas die folgende Zusammensetzung.

| | |
|---|---|
| 58 - 94 Gew.-% vorzugsweise | 73 - 78 Gew.-% SiO₂, |
| 3 - 7 Gew.-% vorzugsweise | 4 - 6 Gew.-% Na₂0, |
| 5 - 14 Gew.-% vorzugsweise | 6 - 12 Gew.-% K₂O |
| 5 - 7 Gew.-% vorzugsweise | 6 Gew.-% CaO. |

Die mit dem erfindungsgemäßen Verfahren hergestellten Zahnersatzstücke aus Glas besitzen ähnliche Eigenschaften wie übliche im Dentalrestaurationsbereich verwendete Keramikstoffe. Im Unterschied zu diesen sind jedoch bei den erfindungsgemäßen Zahnersatzstücken aus Glas keine mehrstufigen Auftrags- und Brennprozesse notwendig. Stattdessen können die erfindungsgemäßen Zahnersatzstücke aus Glas in einem Verfahrensschritt durch Schmelzpressen hergestellt werden. Dies ist erheblich preiswerter. Weiterhin können die Zahnersatzstücke auch durch entsprechenden Pigmentzusatz eingefärbt werden, so daß sie der Zahnfarbe des Patienten angepaßt werden können. Bei Verwendung von ungefärbtem Klarglas hat dies Den Vorteil, daß man on außen durch das Inlay auf den Zahninnenraum schauen kann, was die Überprüfung des Zahnes auf Sekundärkaries oder andere Defekte ermöglicht, ohne daß das Inlay entfernt werden muß.

### Bezugszeichenliste

- 1: Modell
- 2: Aufnahmevorrichtung
- 3: Glasstab mit Verdickung
- 4: Preßstempel
- 5: Schlupfbuchse
- 6: Preßkopf
- 7: Brenner
- 8: Infrarotmeßgerät
- 9: Steuerung

## Patentansprüche

1. Verfahren zur Herstellung von Zahnersatzstücken aus Glas gekennzeichnet durch die folgenden Verfahrensschritte:
a) Anfertigen eines Modells (1) des kranken Zahnes, der mit dem Zahnersatzstück ausgestattet werden soll,
b) Fixieren des Modells (1) in einer Aufnahmevorrichtung (2),
c) Herstellung einer Glasschmelze und Einpressen der Glasschmelze in die Hohlräume des Modells (1),
d) Abkühlen des mit der Glasschmelze gefüllten Modells (1).

2. Verfahren nach dem Anspruch 1, **dadurch gekennzeichnet,** daß die Zahnersatzstücke Füllungen, Inlays, Onlays, Verblendschalen oder Kronen sind.

3. Verfahren nach den Ansprüchen 1 oder 2, **dadurch gekennzeichnet,** daß das Modell vor dem Einpressen der Glasschmelze auf Temperaturen von 200 bis 500° C vorgeheizt wird.

4. Verfahren nach den Ansprüchen 1 bis 3, **dadurch gekennzeichnet,** daß zur Herstellung der Glasschmelze ein Glasstab, ein Glasstab mit Verdickung am unteren Ende oder Haltemittel mit Glas am unteren Ende eingesetzt werden und das am unteren Ende befindliche Glas (3) mit einem Brenner (7) oder einer sonst geeigneten Wärmequelle geschmolzen wird.

5. Verfahren nach den Ansprüchen 1 bis 4, **dadurch gekennzeichnet,** daß das Erreichen des Schmelzpunktes des Glases mit einem Infrarotmeßgerät (8) oder anderen geeigneten Meßgeräten ermittelt wird.

6. Verfahren nach den Ansprüchen 4 oder 5, **dadurch gekennzeichnet,** daß der Glasstab, der Glasstab mit Verdickung am unteren Ende oder das Haltemittel mit Glas am unteren Ende während des Erhitzens des Glases um die eigene Achse rotiert.

7. Verfahren nach den Ansprüchen 1 bis 6, **dadurch gekennzeichnet,** daß das Pressen der Glasschmelze in das Modell (1) mit einem Preßkopf (6) erfolgt, der mit einem Preßstempel (4) versehen ist.

8. Verfahren nach Anspruch 7, **dadurch gekennzeichnet,** daß der Preßstempel (4) auf Temperaturen von 200 bis 500° C vorgeheizt wird.

9. Zahnersatzstücke aus Glas hergestellt nach dem Verfahren gemäß den Ansprüchen 1 bis 8.

10. Zahnersatzstücke nach Anspruch 9, **dadurch gekennzeichnet,** daß es eine Füllung, Inlay, Onlay, eine Verblendung oder eine Krone ist.

11. Zahnersatzstücke nach den Ansprüchen 9 oder 10, **dadurch gekennzeichnet,** daß er aus Silikatglas besteht.

12. Zahnersatzkörper nach Anspruch 11, **dadurch gekennzeichnet,** daß das Silikatglas die folgende Zusammensetzung aufweist
58 - 94 Gew.-% SiO₂,
3 - 7 Gew.-% Na₂0,
5 - 14 Gew.-% K₂O
5 - 7 Gew.-% CaO.

13. Zahnersatzstücke nach Anspruch 12, **dadurch gekennzeichnet,** daß das Silikatglas die folgende Zusammensetzung aufweist
73 - 78 Gew.-% SiO₂
4 - 6 Gew.-% Na₂O
6 - 12 Gew.-% K₂O
6 Gew.-% CaO
